# NEUE EUROPÄISCHE PATENTSCHRIFT

(11) **EP 2 054 112 B2**
(45) Veröffentlichungstag und Bekanntmachung des Hinweises auf die Entscheidung über den Einspruch: **16.06.2021**
(45) Hinweis auf die Patenterteilung: 30.03.2011
(21) Anmeldenummer: 07785091.5
(22) Anmeldetag: 14.08.2007
(51) Int. Cl.: A61M 5/315

(54) **INJEKTIONSVORRICHTUNG MIT KLAUENSICHERUNG**
INJECTION DEVICE WITH CLAW-TYPE SECURING MEANS
DISPOSITIF D'INJECTION DOTÉ D'UNE FIXATION À GRIFFES

(30) Priorität: 14.08.2006 DE 102006038101
(43) Veröffentlichungstag der Anmeldung: 06.05.2009
(73) Patentinhaber: Tecpharma Licensing AG, 3401 Burgdorf (CH)
(72) Erfinder: SCHRUL, Christian, 3400 Burgdorf (CH); TSCHIRREN, Markus, 3422 Kirchberg (CH)
(74) Vertreter: SSM Sandmair
(86) Internationale Anmeldenummer: PCT/CH2007/000396
(87) Internationale Veröffentlichungsnummer: WO 2008/019517

(56) Entgegenhaltungen:
- EP-A- 0 937 471
- EP-A1- 1 855 742
- EP-A1- 1 888 149
- EP-A1- 1 923 085
- WO-A-00/41752
- WO-A-03/053499
- WO-A-2004/006997
- WO-A-2005/072796
- WO-A1-2004/006997
- DE-A1-102006 004 563
- US-A- 5 308 340
- US-A1- 2004 186 441
- US-A1- 2005 261 634

## Beschreibung

Die vorliegende Erfindung bezieht sich auf eine Gewindestange für eine Injektionsvorrichtung beziehungsweise einen Pen, sowie auf eine Klauensicherung, sowie auf eine Injektionsvorrichtung mit einer solchen Gewindestange und/oder einer Klauensicherung.

Wird eine in einer Injektionsvorrichtung zum Beispiel in einer Ampulle enthaltene Substanz vollständig oder teilweise abgegeben, wobei eine zur Verdrängung der Substanz zum Beispiel durch Verschieben eines Stopfens innerhalb der Ampulle vorgesehene auf den Stopfen drückende Gewinde- oder Kolbenstange eingeschoben wird, so kann es dazu kommen, dass diese Kolben- oder Gewindestange wieder versehentlich innerhalb der Injektionsvorrichtung zurückgezogen wird, was zu einer Fehlbedienung der Injektionsvorrichtung führen kann.

Es ist eine Aufgabe der Erfindung eine Gewindestange und eine Injektionsvorrichtung mit einer solchen Gewindestange vorzuschlagen, welche sicherstellen können, dass die Gewindestangen nach dem vollständigen Einschieben nicht mehr herausgezogen werden kann.

Diese Aufgabe wird durch den Gegenstand des Anspruchs 1 gelöst. Vorteilhafte Ausführungsformen ergeben sich aus den abhängigen Ansprüchen.

Eine erfindungsgemäße Gewindestange weist ein Verdrehsicherungselement, insbesondere eine Klauensicherung auf, welche so an der Gewindestange und bevorzugt an deren proximalen Ende angeordnet ist, dass die Verdrehsicherung oder Klauen in diese Verdrehsicherungselemente oder Klauen haltende oder eingreifende Gegenelemente eingeschoben werden können, wenn die Gewinde- oder Kolbenstange bis zu einer vorgegebenen zum Beispiel distalen Endposition innerhalb der Injektionsvorrichtung verschoben wurde. Dabei sind die Klauen oder Verdrehsicherungsgegenelemente, in welche die Klauen oder Verdrehsicherungselemente der Gewindestange eingreifen, vorzugsweise mit der Injektionsvorrichtung oder einem Teil davon, wie zum Beispiel einem Gehäuse der Injektionsvorrichtung, fest verbunden. Eine in die Injektionsvorrichtung eingeschobene Gewindestange kann somit nach dem Einschieben der Klauen oder Verdrehsicherungselemente in die entsprechenden Verdrehsicherungsgegenelement der Injektionsvorrichtung nicht mehr gedreht werden, da durch den Eingriff der Elemente, wie zum Beispiel den Eingriff von vorstehenden Stegen in Nuten, die Gewinde- oder Kolbenstange mit der Injektionsvorrichtung verdrehsicher gekoppelt wird oder ist, was ein Verdrehen unmöglich macht. Eine Gewindestange kann somit in einer eingeschobenen Endposition gehalten werden, da eine Verdrehung aufgrund der Klauensicherung nicht mehr möglich ist und eine axiale Verschiebung aufgrund der Gewindekopplung verhindert wird.

Somit ist bei der Verwendung der erfindungsgemäßen Gewindestange keine weitere Haltevorrichtung in der Injektionsvorrichtung zwingend von Nöten, welche die Gewindestange beispielsweise in der Endposition hält und fixiert. Die Fixierung der Gewindestange wird bereits durch die erfindungsgemäße Gewindestange und die daran ausgebildete oder angeordnete Klauensicherung erreicht.

Die Verdrehsicherungsgegenelemente der Injektionsvorrichtung können als Einbuchtungen in oder Klauen an der Injektionsvorrichtung oder an Teilen der Injektionsvorrichtung, wie der Drehhülse, ausgebildet sein. Es sind zahlreiche Ausgestaltungen der Gegenelemente der Injektionsvorrichtung möglich, wobei die Verdrehsicherungsgegenelemente bevorzugt so ausgebildet sind, dass sie mit den in die Verdrehsicherungsgegenelemente eingreifenden oder eingeschobenen Verdrehsicherungselementen der Gewindestange einen Formschluss bilden, so dass eine Verdrehung der Gewindestange in proximaler und/oder distaler Richtung durch den Formschluss verhindert wird. Die Verdrehsicherungselemente an der Gewindestange können zum Beispiel federnd gelagert sein, um bei beispielsweise in der distalen Endposition in die Verdrehsicherungsgegenelemente der Injektionsvorrichtung einschnappen oder einrasten zu können. Auch können die Verdrehsicherungselemente der Gewindestange fest gelagert sein und zumindest leicht oder teilweise verformbar sein, so dass sie in der distalen Endposition in die Verdrehsichenmgsgegenelemente der Injektionsvorrichtung einschnappen oder einrasten können. Sind die Verdrehsieherungselemente in die Verdrehsicherungsgegenelemente eingeschnappt oder eingefahren, sind sie formschlüssig mit den Verdrehsicherungsgegenelementen verbunden, so dass eine Bewegung oder Drehung der Gewindestange in proximaler und/oder distaler Richtung verhindert werden kann.

Um die Menge einer abzugebenden Substanz an einer Injektionsvorrichtung einzustellen, wird an einem Einstellelement, meistens einem Dosierknopf oder einem Dosierring, eine Einstellbewegung zum Beispiel durch Drehung des Dosierelementes durchgeführt, wobei die Größe der Drehung, also zum Beispiel ein Drehwinkel, definiert, welche Menge der Substanz mit dem nächsten Injektionsvorgang aus der Injektionsvorrichtung abgegeben wird. Bei einer Injektionsvorrichtung, welche eine feste und zum Beispiel voreingestellte Menge einer Substanz abgeben soll, einem so genannten fixed-dose-Pen, wird die Einstellbewegung durchgeführt, um zum Beispiel den Pen zur Abgabe einer vordefinierter Dosis aufzuziehen.

Die Gewindestange der Injektionsvorricbtung ist so ausgebildet, dass diese im Zusammenwirken mit einem oder mehreren zum Beispiel an dem Pen oder dem Gehäuse des Pens angeordneten oder auch an mit dem Einstellelement gekoppelten Eingriffelementen, zum Beispiel elastischen oder radial nach innen oder außen vorgespannten Schnappelementen oder Rastelementen, vorgegebene festgelegte Drehpositionen einnehmen kann, in welchen diese Rastelemente in Eingriffbereiche der Gewindestange eingreifen und so für eine Kopplung zwischen der Gewindestange und dem Pen oder zwischen der Gewindestange und einem Einstellelement sorgen. Hierzu weist die Gewindestange bevorzugt in Längsrichtung der Gewindestange Eingriflbereiche, Nuten oder Rillen auf, welche zum Beispiel auch das Gewinde auf der Außenseite der Gewindestange durchbrechen können, so dass die Gewindestange im Querschnitt zum Beispiel als Stern mit drei, bevorzugt vier oder auch mehr als vier, wie zum Beispiel fünf oder sechs, Zacken oder Stegen ausgebildet ist, so dass eine robuste und einfache Einstellung einer fest vorgegebenen Dosis (fixed-dose) dadurch ermöglicht wird, dass die Gewindestange zum Beispiel bei einer kreuzförmigen Ausgestaltung mit vier Zacken nur zu vier definierten und stabilen Drehpositionen gedreht werden kann. Bei den stabilen Drehpositionen greifen ein oder mehrere Eingriffselemente, welche der konkreten Ausformung der Gewindestange entsprechend zum Beispiel um diese herum angeordnet sind, in die Nuten der Gewindestange ein und halten diese bei sternförmiger Ausbildung zum Beispiel nur in vier definierten Drehpositionen. Eine Drehung der Gewindestange führt somit zum Beispiel nur nach 90°, 180°, 270° und weiteren Vielfachen von 90° zu Stabilen und definierten Drehpositionen der Gewindestange.

Vorzugsweise ist die Gewindestange so ausgebildet, dass zumindest im Bereich der Nuten oder axial verlaufenden Eingriffsbereiche die äußeren oder peripheren Bereiche der Gewindestange eine leichte Abschrägung aufweisen, so dass die bevorzugt radial in Richtung auf die Gewindestange vorgespannten Eingriffselemente, welche vorteilhaft eine Drehung der Gewindestange in eine Richtung durch ein Gleiten über die abgeschrägten Bereiche ermöglichen und eine Drehung in die Sperr- oder Gegenrichtung durch Eingriff in die Eingriffsbereiche sperren, bei einer Drehung der Gewindestange entgegen der Spernichtung leicht außer Eingriff gebracht und aus den Eingriffsbereichen herausgedrückt oder herausgegeben werden können, um durch die Drehbewegung über die abgeschrägten Außenbereiche in den benachbarten Eingriffsbereich geführt zu werden. Eine Abschrägung der Außenbereiche führt dazu, dass ein Zacken oder Steg der Gewindestange zwischen zwei Nuten oder Eingriffsbereichen eine höhere und eine niedrige Flanke hat, wobei die höhere Flanke bevorzugt auf derjenigen Seite angeordnet ist, aus welcher das Eingriffselement nicht mehr herausgedrückt werden soll, um über den abgeschrägten Außenbereich zu gelangen, so dass eine Blockierung der Drehung der Gewindestange durch das Eingriffselement realisiert werden kann. Andererseits wird durch die kleinere Flanke ein Weiterdrehen der Gewindestange vereinfacht, da das Eingriffselement nur den Weg der kleineren Flanke entlang zu dem abgeschrägtern Bereich heraus gedrückt werden muss, um die Drehung der Gewindestange zu ermöglichen. Vorzugsweise sind die Eingriffselemente auch mit einer Schräge versehen, welche beispielsweise korrespondierend zur Schräge des peripheren Bereichs der Gewindestange ausgebildet sein kann und welche ein Herausdrücken des Eingriffselements bei einer Drehbewegung in einer Freigaberichtung vereinfacht oder ermöglicht.

Bevorzugt weist die Gewindestange am proximalen Ende eine Erweiterung auf, wie zum Beispiel einen umlaufenden Ring oder radial abstehenden Steg, von welchem in distale Richtung gerichtet mindestens ein Eingriffselement, wie zum Beispiel ein oder mehrere Stege vorstehend herausragen, um zum Beispiel bei einem Eingriff mit entsprechenden Gegenelementen zum Beispiel nach vollständigem Einschieben der Gewindestange eine Klauensicherung zu realisieren, wobei die Gewindestange so in korrespondierende Gegenelemente oder Klauen eingreift, dass eine weitere Drehbewegung der Gewindestange oder des Dosierelements relativ zur Injektionsvorrichtung blockiert wird und die Injektionsvorrichtung somit nicht mehr verwendet werden kann.

Die Erfindung bezieht sich gemäß einer weiteren Ausführungsform auf eine Injektionsvorrichtung mit einer wie oben beschriebenen Gewindestange.

Vorteilhaft ist an der Injektionsvorrichtung ein Dosierelement, wie zum Beispiel ein Dosierknopf oder ein Drehknopf vorgesehen, welcher mit weiteren Elementen, wie zum Beispiel einer Drehhülse oder einem Drehelement verbunden sein kann. Mittels des Dosierelementes kann die abzugebende Dosis eingestellt, also die Injektionsvorrichtung aufgezogen oder aufdosiert werden. Vorzugsweise wird durch eine Drehbewegung des Dosierelementes ein Federelement, wie zum Beispiel eine Torsionsfeder, gespannt, welche die Energie für die nachfolgende Injektion und den Vorschub der Gewindestange speichert und nach Betätigung eines Auslöseelementes frei gibt. Eine mit dem Dosierelement verbundene Drehhülse oder auch das Dosierelement selber weist bevorzugt mindestens ein und vorteilhaft mindestens zwei zum Beispiel einander gegenüber liegende radial nach innen vorgespannte Eingriffselemente auf, welche in korrespondierende Eingriffsbereiche der Gewindestange eingreifen können und eine Drehung der Gewindestange relativ zur Drehhülse oder zum Dosierelement in eine Richtung ermöglichen. Bei einer Drehung der Drehhülse oder des Dosierelementes in eine Gegenrichtung bleiben die Eingriffselemente im Eingriff mit der Gewindestange und nehmen diese mit, so dass zum Beispiel die in der Torsionsfeder durch die Einstellbewegung gespeicherte Energie in eine Drehbewegung der Gewindestange umgesetzt werden kann.

Vorzugsweise ist an der Drehhülse oder dem Dosierelement auch noch mindestens ein radial nach außen vorgespanntes Eingriffselement vorgesehen, welches zum Beispiel in ein Fenster oder eine Nut oder Ausnehmung der Injektionsvorrichtung eingreifen kann, um so die Drehhülse oder das Dosierelement in einer vorgegebenen Drehposition zum Beispiel nach dem Einstellen der Dosis oder Aufziehen der Injektionsvorrichtung zu halten oder zu arretieren. Das zur Arretierung dienende Halteelement kann zum Beispiel mittels eines Auslöseknopfes wieder freigegeben werden, wobei zum Beispiel nach Betätigung des Auslöseknopfes das radial nach außen vorgespannte Eingriffselement wieder in eine radial nach innen gerichtete Richtung geschoben wird, so dass die Drehhülse oder das Dosierelement nicht mehr mit der Injektionsvorrichtung gekoppelt ist und eine Drehbewegung zum Beispiel durch die Torsionsfeder ermöglicht wird.

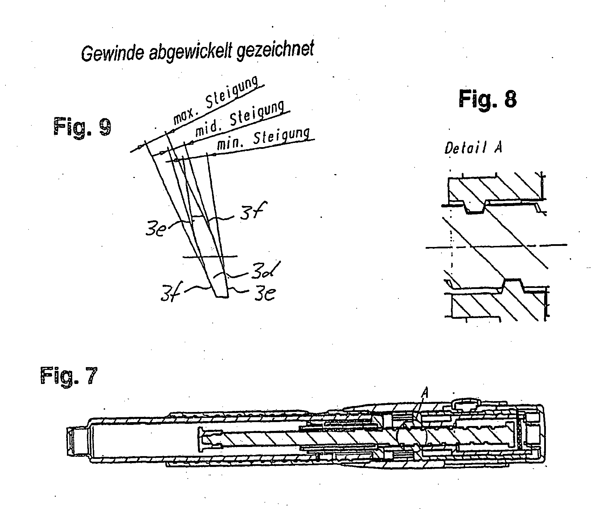

Vorzugsweise weist die Injektionsvorrichtung als Teil der Injektionsvorrichtung integriert und zum Beispiel fest mit der Injektionsvorrichtung verbunden oder als separates Element vorgesehen mindestens ein Führungselement auf, welches ebenfalls mindestens ein elastisches und zum Beispiel radial nach innen vorgespanntes Halteelement aufweist, welches in mindestens einen Eingriffsbereich der Gewindestange eingreifen kann, um zum Beispiel eine Drehbewegung der Gewindestange relativ zur Injektionsvorrichtung in eine Richtung zu ermöglichen und in die Gegenrechtung zu sperren. Bevorzugt weist das Führungselement auch ein Innengewinde auf, welches auch aus einem oder mehreren Teilgewindesegmenten bestehen kann. Dieses Innengewinde oder die Teilgewindesegmente können so ausgebildet sein, dass diese mehrere Anlageflanken haben, wie beispielsweise bei dem in Figur 9 abgewickelt gezeichneten Gewinde ersichtlich, wodurch ein Gewindeeingriff für Gewinde unterschiedlicher Steigungen ermöglicht wird. Beispielsweise können die Gewindesegmente so ausgebildet sein, dass verschiedene Gewindestangen mit einem Außengewinde unterschiedlicher Steigung zwischen einer durch erste Anlageflanken des Innengewindes definierten minimalen Steigung und durch zweite Anlageflanken des Innengewinde definierten maximalen Steigung sicher gefübrt werden können. Hierdurch ist es zum Beispiel möglich, dass unterschiedliche Dosen durch die gleiche Drehbewegung je nach zu verabreichender Substanz dadurch eingestellt werden können, dass Gewindestangen mit unterschiedlicher Steigung des Außengewindes verwendet werden.

Bevorzugt weist die Injektionsvorrichtung Eingriffselemente oder Klauen auf, um mit entsprechenden Eingriffselementen oder Klauen der Gewindestange eine Klauenkupplung zu realisieren. Die Eingriffselemente der Injektionsvorrichtung können zum Beispiel an einer in proximale Richtung weisenden Oberfläche einer Drehhülse, einer Führungshülse oder der Injektionsvorrichtung selbst vorgesehen sein.

Die Injektionsvorrichtung kann ein Übertragungselement aufweisen welches mit einem Ausschüttelement, wie zum Beispiel einer Kolbenstange oder einer Gewindestange, der Injektionsvorrichtung gekoppelt ist und welches mit einem Anzeigeelement, welches zum Beispiel mit einer Ampulle, welche in die Injektionsvorrichtung eingesetzt werden kann, gekoppelt werden kann.

Bei der Verwendung von Injektionsvorrichtungen ist es vorteilhaft, wenn eine Anzeige vorgesehen ist, an welcher Daten bezüglich der bereits verabreichten Dosen oder der noch in der Injektionsvorrichtung enthaltenen Dosen oder bezüglich des aktuellen Ausschüttvorganges abgelesen werden können. Jedoch können Probleme auftreten, wenn diese Anzeige aufgrund eines Fehlers nicht richtig funktioniert, da ein Benutzer zum Beispiel fälschlicherweise annimmt, dass noch eine größere als tatsächlich vorhandene Substanzmenge in der Injektionsvorrichtung enthalten ist.

Ein Anzeigelement zum Anzeigen eines Verabretchungsparameters, wie zum Beispiel einer in einer Injektionsvorrichtung noch enthaltenen oder bereits aus der Injektionsvorrichtung, wie zum Beispiel aus einer in der Injektionsvorrichtung eingesetzten Ampulle, ausgeschütteten Menge einer Substanz ist möglichst unmittelbar und bevorzugt unmittelbar mit einem Ausschüttelement der Injektionsvorrichtung, also zum Beispiel einer Kolbenstange oder Gewindestange, welche zum Beispiel für einen Vorschub zum Beispiel eines Stopfens in eine Ampulle oder in einem Reservoir sorgen, gekoppelt Durch die möglichst unmittelbare Kopplung des Anzeigelementes mit dem Vorschubelement können Fehler bei der Anzeige weitgehend ausgeschlossen werden, da keine oder nur wenige Fehler verursachende oder fehleranfällige Zwischenelemente vorgesehen sind. Somit kann eine robuste und zuverlässige unmittelbare Anzeige geschaffen werden, welche beispielsweise als Restmengenanzeige oder Echtzeitanzeige verwendet werden kann. Das Anzeigeelement kann als Verabreichungsparameter auch die Uhrzeit der Abgabe oder die Ausschüttzeit beispielsweise zusätzlich anzeigen, um einem Benutzer zu ermöglichen, den Abgabezeitpunkt überprüfen zu können oder um den Abgabezeitpunkt zum Beispiel speichern und für Auswertungszwecke verwenden zu können.

Vorzugsweise ist das Anzeigelement unmittelbar mit dem Ausschüttelement verbunden, also zum Beispiel relativ zu diesem verschiebe- und verdrehsichere gelagert, und weist zum Beispiel an einer Außenseite in Umfangrichtung und/oder Längsrichtung eine Markierung zur Dosisanzeige auf, welche beispielsweise an einem Fenster oder mittels einer Markierung, an welcher sich das Anzeigelement durch Drehen und Verschieben vorbei bewegt, abgelesen werden kann.

Alternativ ist das Anzeigelement mit dem Vorschub- oder Ausschüttelement gekoppelt, also nicht unmittelbar mit diesem verbunden, wobei die Kopplung beispielsweise durch einen Gewindeeingriff oder einen anderen Bewegung oder eine Kraft übertragenden Mechanismus, wie zum Beispiel eine Schnecke, ein Zahnrad oder ein Getriebe, realisiert sein kann. Beispielsweise kann das Anzeigeelement ein Gewinde und bei der Ausbildung eines Anzeigeelementes als Hülse ein Innengewinde aufweisen, welches in ein Außengewinde einer Kolbenstange oder Gewindestange eingreift, so dass das zum Beispiel axial verschiebesicher aber drehbar in der Injektionsvorrichtung gelagerte Anzeigeelement unmittelbar durch eine Drehung oder Verschiebung der Gewindestange oder Kolbenstange gedreht wird, wobei an Hand der Drehung zum Beispiel durch Beschriftung auf der Außenseite des Anzeigelementes an einer Skalierung, welche sich beispielsweise nicht mitdreht, abgelesen werden kann, welche Dosis abgegeben wurde oder noch vorhanden ist. Dabei ist im Falle eines Gewindeeingriffes das Gewinde vorteilhaft selbsthemmungsfrei ausgebildet, so dass ein einfaches Drehen des Anzeigeelementes ermöglicht wird und das Anzeigeelement zu keiner Behinderung zum Beispiel einer Aufdosier- oder Ausschüttbewegung führt.

Vorteilhaft kann das Anzeigeelement nicht an einer Injektionsvorrichtung, sondern an einer in die Injektionsvorrichtung emzusetzenden Ampulle vorgesehen sein, welche erst bei oder nach dem Einsetzen in die Injektionsvorrichtung so mit zum Beispiel einem Koppelelement der Injektionsvorrichtung gekoppelt wird, dass eine Bewegung zum Beispiel eines Stopfens der Ampulle in eine entsprechende Bewegung des Anzeigeelementes umgesetzt wird, wodurch eine Echtzeitanzeige realisiert werden kann.

Die Injektionsvoniehtung weist vorteilhaft mindestens eine Durchbrechung, wie zum Beispiel ein Sichtfenster, auf, an welchem zum Beispiel eine auf der Außenseite des Anzeigelementes angebrachte Markierung des in der Injektionsvorrichtung vorgesehenen Anzeigeelementes abgelesen werden kann.

Zur axial verschiebesicheren aber drehbaren Lagerung des Anzeigeelement kann zum Beispiel eine Ringnut oder ein Ringsteg an der Injektionsvorrichtung vorgesehen sein, in welche ein entsprechendes Gegenelement, also zum Beispiel ein Ringsteg oder eine Ringnut der Anzeigeelementes, eingreift.

Die Injektionsvorrichtung kann so ausgebildet sein, dass das Anzeigeelement innerhalb der Injektionsvorrichtung, zum Beispiel bei dem Einsetzen einer Ampulle, verschiebbar ist, wobei zum Beispiel die Anzeige des Anzeigeelementes nicht an einem Fenster ablesbar ist, wenn keine Ampulle eingesetzt ist und beispielsweise ein Farbcode an der Umfangsseite des Anzeigeelementes anzeigt, dass eine Ampulle fehlt. Erst bei oder nach Einsetzen der Ampulle wird zum Beispiel durch den Ampullen-Einsetzvorgang das Anzeigeelement so relativ zu einer Ableseposition, wie zum Beispiel einem Sichtfenster, verschoben, dass die Anzeige oder eine Beschriftung des Anzeigeelementes erkennbar wird. Dabei kann sich das Anzeigeelement auch vollständig oder zum Teil innerhalb der Ampulle befinden.

Die Injektionsvorrichtung kann ein Verfahren zum Sichern einer Mechanik umfassen, insbesondere zum Sichern einer Einstellmechanik oder eines Einstellelements einer Injektionsvorrichtung eine Betätigung, wie zum Beispiel eine Sicherung vor Verdrehung des Einstellelements, wobei die Betätigungs- oder Verdrehsicherung durch ein Einschieben einer Ampulle in die Injektionsvorrichtung aufgehoben werden kann.

Wenn eine Injektionsvorrichtung verwendet wird, in welche eine Ampulle vor dem Gebrauch der Injektionsvorrichtung eingesetzt wird, wie zum Beispiel eine unmittelbar vor dem Gebrauch einzusetzende und abzumischende 2-Kammer-Ampulle, kann es zu Problemen kommen, wenn ein Benutzer der Injektionsvorrichtung schon vor dem Einsetzen der Ampulle einen Einstell- oder Bedienvorgang durchführt.

Eine Injektionsvorrichtung weist ein Gehäuse und ein in dem Gehäuse gelagertes oder mit dem Gehäuse verbundenes oder gekoppeltes Bedienelement auf, wobei das Bedienelement, wie zum Beispiel ein Einstell-, Druck- oder Drehknopf, so in dem Gehäuse gelagert oder mit dem Gehäuse gekoppelt oder verbunden ist, dass das Bedienelement von einer ersten Halteverbindung in einer ersten Position im Bezug auf das Gehäuse der Injektionsvorrichtung gehalten, also zum Beispiel gegen eine axiale Verschiebung gesichert, werden kann. Dabei ist die Halteverbindung so ausgebildet, dass diese während und bevorzugt nach dem Einbringen oder Einsetzen oder Einschieben einer Ampulle gelöst wird, so dass das Bedienelement nach dem Einbringen der Ampulle in eine zweite bevorzugt axial in proximale Richtung zur ersten Halteposition versetzte Halteposition verschoben wird und dort von einer zweiten Halteverbindung gehalten wird. Dabei wird vorzugsweise das Bedienelement durch das Einbringen oder Einschieben der Ampulle ebenfalls relativ zum Gehäuse der Injektionsvorrichtung verschoben, also zum Beispiel in proximale Richtung aus der Injektionsvorrichtung herausgeschoben. Jedoch kann die Injektionsvorrichtung auch so aufgebaut sein, dass eine Kupplung oder ein Koppelelement vorgesehen ist, welches durch das Einschieben der Ampulle verschoben wird, indem es zum Beispiel in Anlage mit einem proximalen Ampullenrand kommt, und so das Bedienelement freigibt. Dabei kann das Bedienelement selbst relativ zur Injektionsvorrichtung still stehen oder ebenfalls mit verschoben werden.

Vorzugsweise ist das Bedienelement oder Koppelelement so in der Injektionsvorrichtung gelagert, dass erst nach dem vollständigen Einsetzen oder Einschieben oder Eindrehen einer Ampulle, was mit dem Abmischen von zum Beispiel zwei in der Ampulle enthaltenen Substanzen einhergehen kann, aus der ersten Halteverbindung in die zweite Halteverbindung verschoben wird. Beispielsweise kann das Bedien- oder Koppelelement so in der Injektionsvorrichtung vorgesehen sein, dass eine einzubringende oder einzuschiebende Ampulle mit einem zum Beispiel vorher bekannten Maß oder Abmessungen erst auf den letzten Teil der Einschubstrecke mit dem Koppel- oder Bedienelement in Berührung kommt, so dass die Ampulle vor dieser letzten Wegstrecke in die Injektionsvorrichtung zum Beispiel eingeschraubt werden kann, ohne das Koppel- oder Bedienelement zu berühren bzw. zu verschieben und erst die nachfolgende Berührung der Ampulle mit dem Koppel- oder Bedienelement und die Verschiebung durch das dann vollständige Einschieben der Ampulle dazu führt, dass das Koppelelement die Mechanik zur Bedienung der Injektionsvorrichtung entriegelt oder dass das Bedienelement zum Beispiel durch Ausfahren aus dem Gehäuse der Injektionsvorrichtung für die Bedienung und zum Beispiel Einstellung durch einen Benutzer freigegeben wird.

Die erste und/oder zweite Halteverbindung können zum Beispiel durch eine Rastverbindung realisiert werden, wobei beispielsweise ein Rastring vorgesehen sein kann, welcher von einem Koppel- oder Bedienelement radial nach ihnen oder nach außen ragt und zusammen mit einer Ringnut der Injektionsvorrichtung oder des Gehäuses eine erste Halteverbindung bildet und einer weiteren Ringnut der Injektionsvorrichtung oder des Gehäuses die zweite Rastverbindung bildet. Ebenso kann das Halteelement auch durch andere mechanische Koppelungen realisiert werden, welche bevorzugt lösbar sind, wenn eine bestimmte Mindestkraft auf diese Kopplung einwirkt.

Die Mechaniksicherung kann zum Beispiel dadurch realisiert werden, dass eine Verdrehsicherung des Einstellelements zum Beispiel durch Führung von vorstehenden Nuten des Einstellelementes vorgesehen ist, so dass das Einstellelement in einer distalen Position gegen eine Verdrehung gesichert ist. Nach dem Eindrehen einer Ampulle wird das verdrehgesicherte Einstellelement so weit in proximale Richtung geschoben, dass die zur Verdrehsicherung dienenden Nuten aus dem diese Nuten haltenden und führenden Elementen zum Beispiel der Injektionsvorrichtung oder des Gehäuses herausgeschoben werden und somit eine Drehung und damit Betätigung des Einstellelementes möglich ist. Ebenso kann auch ein Koppelelement vorgesehen sein, welches eine Drehbewegung des Einstellelementes erst nach einer Verschiebung in proximale Richtung freigibt. Beispielsweise kann dieses Koppelelement ringförmig aufgebaut sein und nach innen und außen weisende Stege haben, welche in Nuten des Einstellelements und Nuten der Injektionsvorrichtung bzw. des Gehäuses eingreifen und so eine Verdrehung des Einstellelementes gegen das Gehäuse der Injektionsvorrichtung verhindern. Wird das Einstellelement durch eine in die Injektionsvorrichtung eingebrachte und zum Beispiel eingedrehte Ampulle zum Beispiel gegen die Kraft einer das Koppelelement in distale Richtung vorspannende Feder verschoben, bis die Stege des Koppelelementes aus den Nuten des Einstellelements und/oder aus den Nuten der Injektionsvorrichtung herausgeschoben werden, so wird die Kopplung der Einstellvorrichtung mit der Injektionsvorrichtung gelöst und die Injektionsvorrichtung kann nach dem bevorzugt vollständigen Einschieben der Ampulle in die Injektionsvorrichtung betätigt werden.

Wenn Injektionsgeräte und insbesondere Injektionsvorrichtungen zur dosierten Abgabe einer Substanz hergestellt werden, so ist deren Aufbau und insbesondere die Dosiermechanik auf einen vorgegebenen Anwendungsfall kanfiguriert. Zum Beispiel wird zur Abgabe einer großen Substanzmenge oder zur Bereitstellung eines großen Hubes des Einstellelementes eine große Steigung eines Innengewindes der Injektionsvorrichtung vorgesehen, in welchem eine Gewindestange oder ein Einstellelement geführt werden. Soll eine einmal entwickelte Injektionsvorrichtung auch für die Abgabe einer zum Beispiel niedriger zu dosierenden Substanz verwendet werden, so muss diese neu konzipiert und hergestellt werden, um zum Beispiel ein Innengewinde mit kleinerer Steigung vorzusehen.

Eine Injektionsvorrichtung weist ein Innengewinde zur Führung zum Beispiel einer Gewindestange oder eines Einstellelementes auf, wobei das Innengewinde so ausgebildet ist, dass es mehrere Anlageflanken aufweist, um verschiedene Gewindestangen mit einem Außengewinde unterschiedlicher Steigung führen zu können, ohne dass das Innengewinde der Injektionsvorrichtung ausgewechselt oder verändert werden muss. Dabei ist das Innengewinde vorzugsweise aus einzelnen Gewindeabschnitten aufgebaut. Diese Gewindeabschnitte können in Umfangsrichtung zueinander versetzt sein und sich zum Beispiel über 1/N-tel des Umfanges erstrecken, wobei N eine natürliche Zahl ist. Beispielsweise sind die Gewindeabschnitte so ausgebildet, dass sie sich über eine halben, einen drittel oder einen viertel Umfang an der Innenseite der Injektionsvorrichtung oder eines Gehäuses davon erstrecken. Die einzelnen Gewindeabschnitte weisen dabei erfindungsgemäß mindestens zwei Anlageflanken auf, an welchen Gewinde unterschiedlicher Steigung geführt werden können.

Vorzugsweise besitzen die Gewindeabschnitte mindestens vier Seitenflächen, in welchen ein Gewinde geführt werden kann, wobei jeweils zwei Seitenflächen zueinander parallel sind. Dabei wechseln sich in Umfangsrichtung, das heißt bei einem Umlauf um ein Gewindesegment oder Gewindeteilelement, die Anlageflächen zur Führung der verschiedenen Gewinde mittelbar oder unmittelbar ab, das heißt die Anlageflächen können entweder unmittelbar aneinander stoßen oder durch Gewindeabschnittssegmente oder Gewindeabschnittsteilstücke, welche zum Beispiel zur Führung weiterer Gewinde unterschiedlicher Steigung dienen, verwendet werden.

Es ist möglich, dass ein solches Gewinde oder Gewindeteilstück so aufgebaut ist, dass mehr als zwei Gewinde unterschiedlicher Steigung geführt werden können. Hierzu kann das Gewinde oder die Gewindesegmenteanlageflächen zur Führung von zum Beispiel Gewindestangen mit einer variablen Steigung zwischen einer durch die Gewindesegmente vorgegebenen minimalen Steigung und einer maximalen Steigung haben. Es ist auch möglich, dass die Gewindesegmente mehr Anlageflächen besitzen und in Umfangsrichtung so angeordnet sind, dass zum Beispiel nur Gewinde mit definierten Steigungen, wie zum Beispiel drei verschiedenen vorgegebenen Steigungen geführt werden können.

Allgemein kann in Abhängigkeit von der Ausbildung eines Gewindesegmentes, insbesondere der Ausbildung der Anlageflächen des Gewindesegments, und in Abhängigkeit von der Verteilung der Gewindesegmente in Umfangsrichtung vorgegeben werden, welche Außengewinde durch solche ein oder mehrere Innengewinde unterschiedlicher Steigung realisierende Gewindesegmente geführt werden können.

Somit können in einer einzigen Injektionsvorrichtung bzw. in einem einzigen ausgebildeten Innengewinde Elemente mit einem Außengewinde unterschiedlicher Steigung geführt werden, ohne dass das Innengewinde oder die Injektionsvorrichtung konstruktiv verändert werden muss. Somit kann die gleiche Injektionsvorrichtung für unterschiedliche Anwendungen verwendet werden, bei welchen zur Dosierung zum Beispiel kleine oder auch große Hübe auszuführen sind.

Die Erfindung wird nachfolgend anhand von Ausführungsbeispielen beschrieben. Es zeigen:
- Figur 1: eine Ausführungsform einer Injektionsvorrichtung in Draufsicht;
- Figur 2: eine perspektivische Querschnittsansicht der Injektionsvorrichtung entlang des Schnittes A-A in Figur 1;
- Figur 3: eine Schnittbildansicht bei einer alternativen Ausführungsform entlang des Schnittes A-A von Figur 1 zusammen mit weiteren Schnitten B-B und C-C der Schnittbildansicht;
- Figur 4: die Injektionsvorrichtung der Figur 3 nach dem Abmischen des Pens und herausgeschobener Mechanik;
- Figur 5: die Injektionsvorrichtung gemäß Figur 4 nach Einstellen der Dosis und Spannen der Feder;
- Figur 6: die Injektionsvorrichtung gemäß Figur 5 nach Ausschütten der Dosis bei blockierter Mechanik und entspannter Feder;
- Figur 7: eine Querschnittsansicht einer Injektionsvorrichtung ohne eingesetzte Ampulle;
- Figur 8: das Detail A in Figur 7 zur Veranschaulichung des Gewindeeingriffes der Führungshülse in die Gewindestange;
- Figur 9: das abgewickelte Gewinde der Führungshülse gemäß Figur 8 mit unterschiedlichen Anlageflanken zur Führung verschiedener Gewindestangen mit einem Außengewinde unterschiedlicher Steigung;

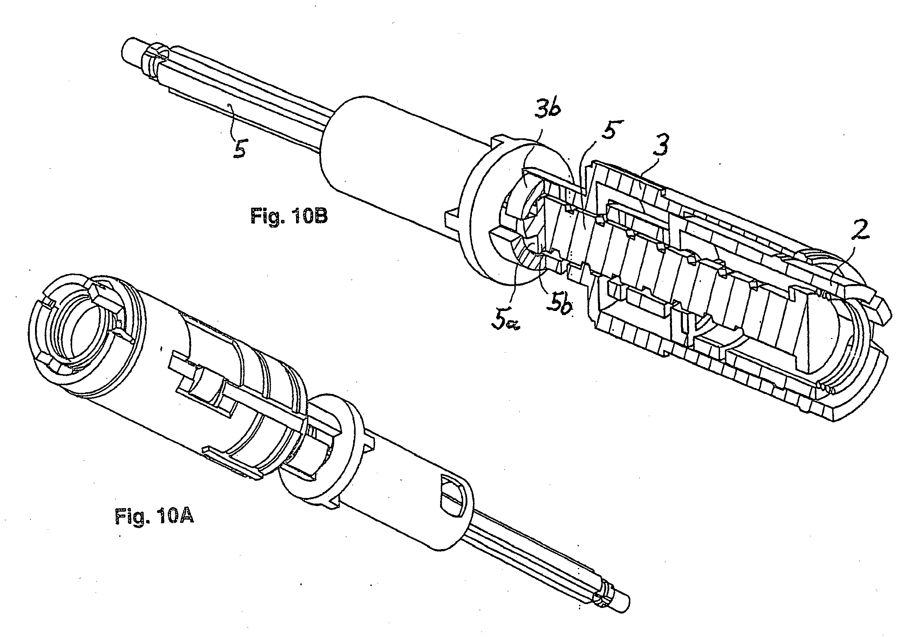

- Figur 10A: die Dosiermechanik der Injektionsvorrichtung in perspektivischer Ansicht;
- Figur 10B: die in Figur 10A gezeigte Dosiermechanik mit einer Querschnittsansicht des proximalen Teiles;
- Figuren 11A- 11C: verschiedene Ausführungsformen einer Echtzeit- oder Restemengenanzeige;
- Figuren 12A - 12B: eine Ausführungsform einer erfindungsgemäßen Klauensicherung; und
- Figuren 13A- 13G: eine Ausführungsform einer erfindungsgemäßen Mechanikverriegelung

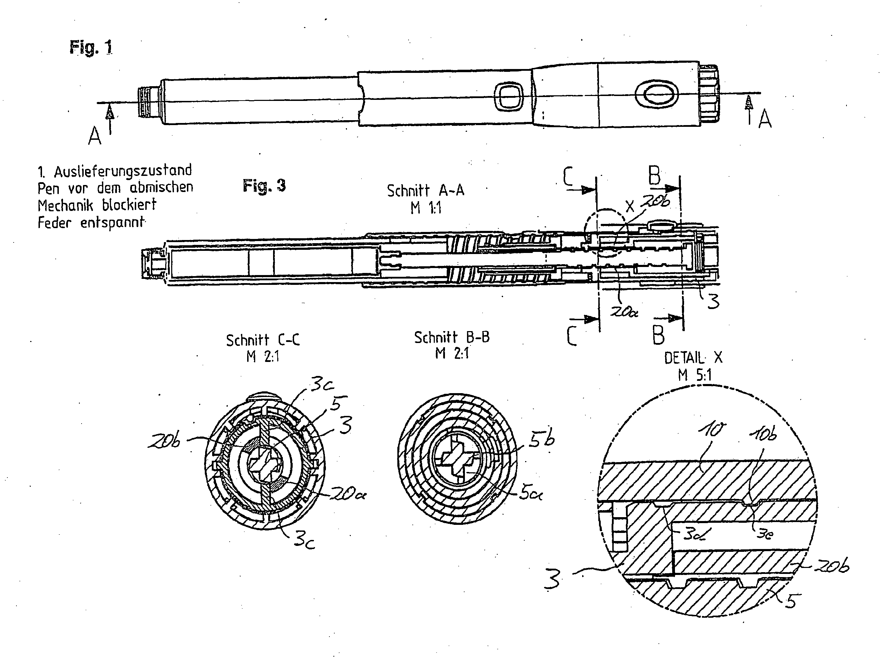

Fig. 1 zeigt einen fixed-dose-Pen, wobei die auszuschüttende Dosis am Dosierknopf 1 einstellbar ist.

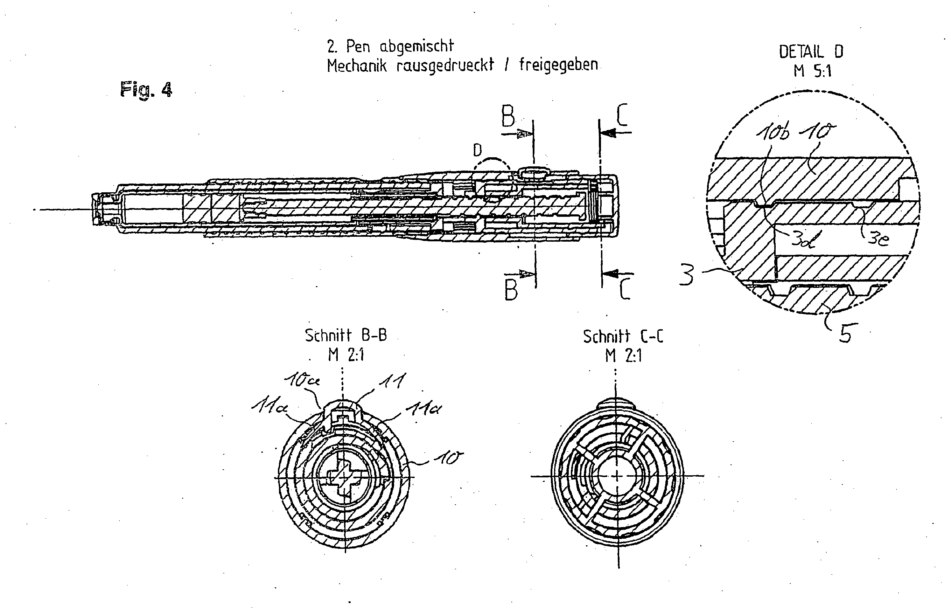

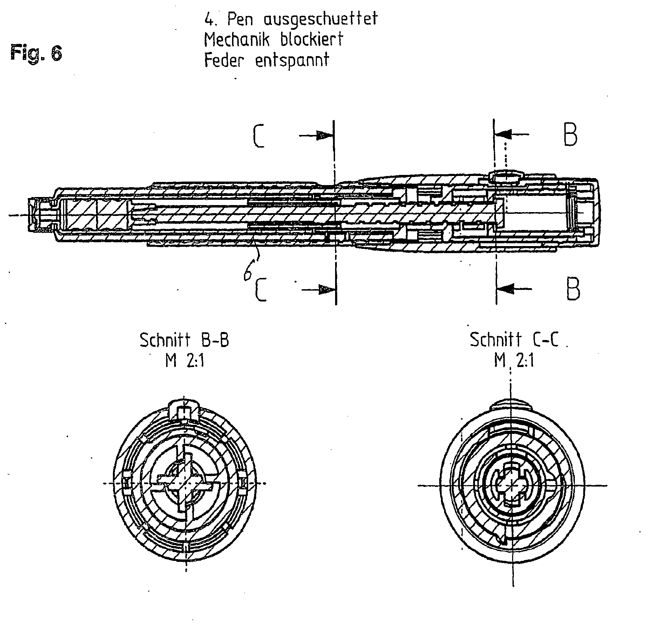

Der Pen weist eine Gewindestange 5 auf, welche wie in den Schnitten der Figuren 3 bis 6 gezeigt im Querschnitt als Stern vier Zacken 5b aufweist, wodurch eine robuste und einfache Einstellung der fixed-dose möglich ist.

Es ist prinzipiell möglich, dass die Gewindestange 5 im Querschnitt sternförmig (Schweizer Kreuz-förmig) ausgebildet sein kann, jedoch kann der Stern auch mehr oder weniger Zacken 5b als das Schweizer Kreuz aufweisen.

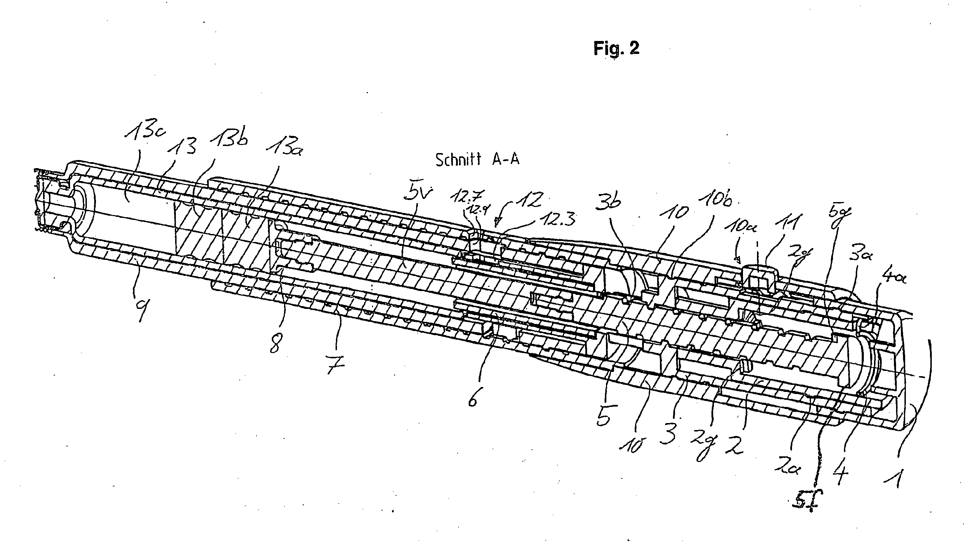

Fig. 2 zeigt den Pen gemäß Fig. 1 in perspektivischer Ansicht im Schnitt A-A.

Die Führungshülse 3 kann mit dem Gehäuse 10 verbunden sein und ist relativ zu dem Gehäuse 10 verdrehsicher gelagert. Innerhalb der Führungshülse 3 ist die Drehhülse 2 mittels einer Schnappwulst 2a drehbar, aber axial nicht verschiebbar, gelagert. Auf der Außenseite der Führungshülse 3 ist der Dosierknopf 1 ebenfalls mittels einer Schnappwulst 3a drehbar, aber nicht axial verschiebbar, gelagert. An der proximalen Seite der Drehhülse 2 und verbunden mit der Führungshülse 3 und der Drehhülse 2 befindet sich ein Federelement 4, in einer bevorzugten Ausführungsform als zwei- bis dreimal gewickelter Federdraht oder Federband, so dass, wenn die Drehhülse 2 relativ zur Führungshülse 3 gedreht wird, die Feder 4, welche zum Beispiel durch Aufbiegungen 4a an den entgegengesetzten Enden einmal mit der Führungshülse 3 und einmal mit der Drehhülse 2 verbunden ist, gespannt wird und so für eine Rückstellkraft gegen die EinstellDrehbewegung sorgt.

Der Dosierknopf 1 wird bevorzugt axial nicht herausgezogen, sondern nur gedreht. Auf der Innenseite des Dosierknopfes 1 befinden sich in axiale Richtung weisend vier Stege, welche in entsprechende Nuten der Drehhülse 2 eingreifen und somit den Dosierknopf 1 mit der Drehhülse 2 so koppeln, dass eine Drehbewegung des Dosierknopfes 1 in eine Drehbewegung der Drehhülse 2 übertragen werden kann. Prinzipiell könnten Dosierknopf 1 und Drehhülse 2 auch einstückig oder als ein Element ausgebildet sein.

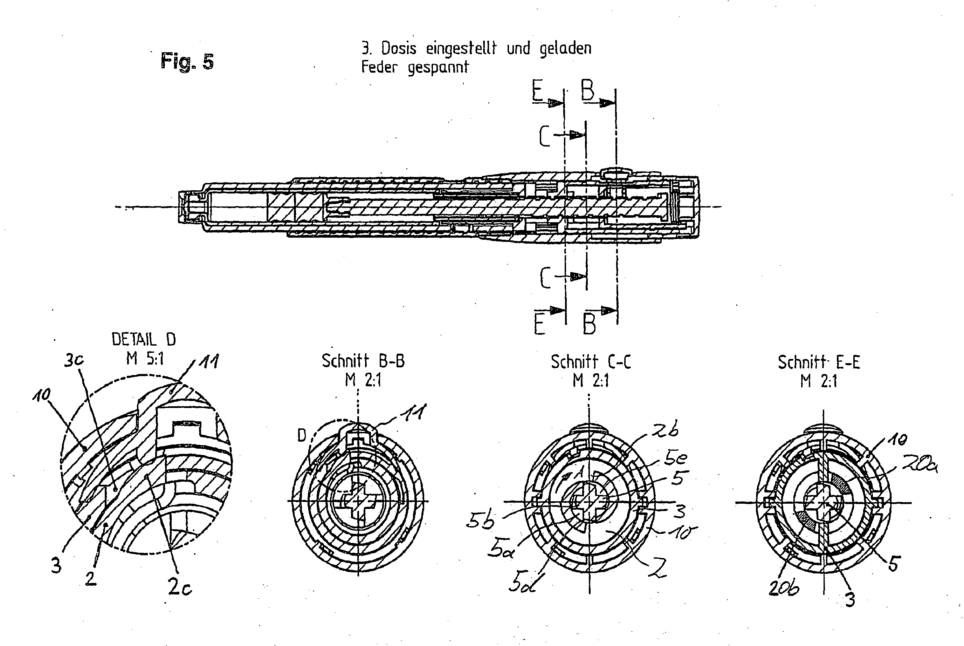

Die Drehhülse 2 weist radial nach innen vorgespannte Schnappelemente 2b, im Ausführungsbeispiel der Fig. 5 im Schnitt C-C gezeigt zwei gegenüberliegende radial nach innen vorgespannte Schnappelemente 2b, auf. Diese Schnappelemente 2b greifen in die vier Nuten 5a der Gewindestange 5 während der Aufziehbewegung ein oder werden an den Zacken 5b vorbeigedreht. Während des Aufziehvorganges ist die Gewindestange 5 verdrehgesichert gelagert, indem sie von einem Schnappelement 3b der Führungshülse 3 gehalten wird. Die Drehhülse 2 weist ein radial nach außen vorgespanntes Schnappelement 2c auf, welches in ein in Figur 5 im Schnitt B-B gezeigtes Fenster oder eine Durchbrechung 3c der Führungshülse 3 gedreht wird, wenn der Aufziehvorgang beendet ist, und verrastet in diesem Fenster 3c so, dass ein Zurückdrehen der Drehhülse 2 relativ zur und innerhalb der Führungshülse 3 aufgrund der Kraft der durch den Einstellvorgang vorgespannten Feder 4 nicht möglich ist. Wird der über dem Fenster 3c der Führungshülse 3 liegende Auslöseknopf 11 des Pens gedrückt, wird das radial nach außen vorgespannte Schnappelement 2c der Drehhülse 2 aus dem Fenster 3c der Führungshülse 3 herausgedrückt und somit die Drehhülse 2 relativ zur Führungshülse 3 freigegeben, so dass diese aufgrund der Kraft der vorgespannten Torsionsfeder 4 um den Einstellweg zurückgedreht werden kann.

Auf der distalen Seite der Drehhülse 2 sind in Umfangsrichtung gegenüberliegend zwei in axialer Richtung vorstehende Anschläge, Stege oder Nocken 2a, 2b vorgesehen, welche eine maximale Drehung der Drehhülse 2 von ca. 110° ermöglichen, da diese Nocken dann in Anlage mit ebenfalls in radialer Richtung, allerdings in proximale Richtung gerichtete, Nocken 3c der Führungshülse 3 kommen.

Beim Zurückdrehen in der im Schnitt C-C der Fig. 5 durch den Pfeil A gezeigten Ausschüttrichtung rasten die radial nach innen vorgespannten Nocken 2b der Drehhülse 2 in den in axialer Richtung verlaufenden Nuten 5a der Gewindestange 5, so dass die Gewindestange 5 von der Rückdrehbewegung der Drehhülse 2 mitgenommen wird und sich somit durch ein Innengewinde 3d der Führungshülse 3 geführt in distale Richtung in den Pen hineinschraubt. Dies bewirkt ein Vorschieben des oder der Stopfen 13a, 13b der Ampulle 13 durch den auf der distalen Seite der Gewindestange 5 oder eines damit verbundenen Verlängerungselements 5v vorgesehenen Stempel 8 in die Ampulle 13 hinein und somit eine Ausschüttung der in der Ampulle 13 enthaltenen Substanz 13c.

Nach erfolgter Ausschüttung kann der Pen durch Drehung des Dosierknopfes 1 wieder neu aufgezogen werden und die gleiche Dosis anschließend ausgeschüttet werden.

Die Gewindestange 5 kann im Spritzgussverfahren mit zwei Formhälften einfach hergestellt werden, wenn die Gewindestange 5 im Querschnitt ein einfaches Kreuz bildet. In axialer Richtung der Gewindestange 5 können die Nuten 5a, in welche der radial nach innen vorgespannte Schnapper 2b der Führungshülse 2 eingreift, durchgängig ausgebildet sein und unterbrechen so das Gewinde 5c auf der Außenseite der Gewindestange 5. Somit können hohe oder steile Flanken für den Schnapper 2b erzielt werden.

Die äußeren oder peripheren Bereiche der Gewindestange 5 weisen eine Schräge 5d auf, so dass der radial nach innen vorgespannte Schnapper 2b der Drehhülse 2 leichter beim Aufziehen darüber hinweg gleiten kann. Hierdurch ergibt sich auch eine höhere Flanke 5e auf der Seite der in axialer Richtung durchlaufenden Nut 5a der Gewindestange 5, in welcher der radial nach innen vorgespannte Schnapper 2b der Drehhülse 2 eingreift, wodurch ein Zurückdrehen der Drehhülse 2 relativ zur Gewindestange 5 sicher verhindert werden kann.

Prinzipiell ist es bei einer solchen Ausgestaltung der Gewindestange 5 relativ einfach möglich, die Gewindesteigung im Fertigungsprozess zu verändern, so dass bei gleicher Einstelldrehbewegung von zum Beispiel 110° unterschiedliche abzugebende Dosismengen in Abhängigkeit von der jeweils konkret vorliegenden Gewindesteigung realisiert werden können. Hierbei kann entweder das Innengewinde 3d der Führungshülse 3 korrespondierend zur geänderten Steigung des Außengewindes 5c der Gewindestange 5 geändert werden. Alternativ ist es auch möglich, dass das Innengewinde 3d der Führungshülse 3 so ausgebildet ist, dass verschiedene Steigungen des zu führenden Außengewindes 5c der Gewindestange 5 geführt werden können, und zwar vollständig in einem Bereich von einer minimalen durch das Innengewinde 3d vorgegebenen Steigung bis zu einer maximalen durch das Innengewinde 3d vorgegebenen Steigung. Figur 9 zeigt einen einzigen Gewindegang des Innengewindes 3d der Führungshülse 3 in aufgerollter Darstellung mit einer minimalen und einer maximalen Steigung, welche sich durch Anlagekanten 3e und 3f des Innengewindes 3d ergeben.

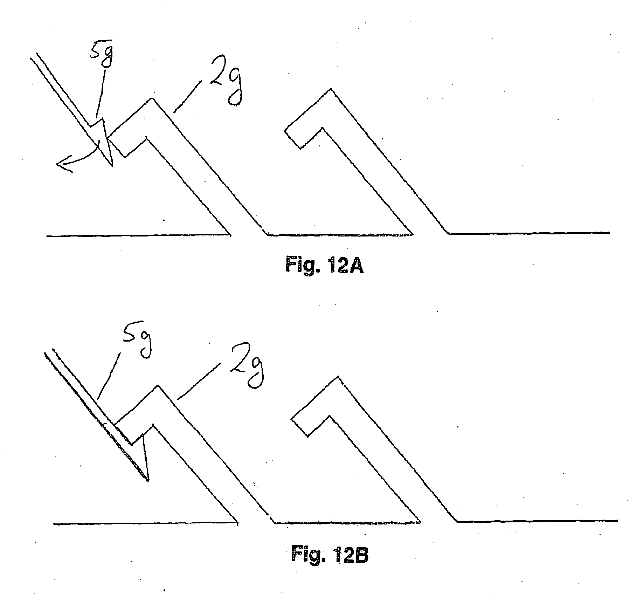

Um sicherzustellen, dass nach dem Ausschütten der letzten Dosis der Pen nicht mehr aufgezogen werden kann, ist an der Gewindestange 5 eine Klauensicherung vorgesehen. Diese weist an der proximalen Seite der Gewindestange 5 eine Erweiterung 5f auf, von welcher in axialer Richtung nach distal weisend zum Beispiel vier Stege 5g vorstehen, welche nach dem Ausschütten der letzten Dosis in entsprechende Gegenanschläge 2g der Drehhülse 2 einfahren oder in diese eingeschoben werden. Die Gewindestange 5 wird dabei axial so weit in die Drehhülse 2 hineinbewegt, dass die Klauen oder Stege 5g der Gewindestange 5 an den entsprechenden Gegenanschlägen 2g der Drehhülse 2 anliegen. Die Stege der Klauensicherung können in die entsprechenden Gegenanschläge 2g einfahren, indem sie beispielsweise bei einer Betätigung der Injektionsvorrichtung zumindest leicht oder teilweise mechanisch verformt oder komprimiert werden und sich zum Beispiel bei Erreichen der Endposition entspannen und in die beispielsweise als Ausnehmungen ausgebildeten Gegenanschläge einfahren. Die Klauen der Klauensicherung können auch federnd gelagert sein. Wird die Injektionsvorrichtung betätigt, gleiten die federnd gelagerten Klauen 5g, wie in Figur 12A gezeigt, an dem Gegenanschlag 2g entlang, indem sie in Pfeilrichtung ausgelenkt werden. Nach Abgabe der Dosis schnappen die Klauen 5g, wie in Figur 12B gezeigt, in den Gegenanschlag 2g ein, so dass die Injektionsvorrichtung nicht mehr aufgezogen werden kann. Unabhängig von der Ausgestaltung der Klauensicherung 5g und der Gegenanschläge 2g wird durch die erfindungsgemäße Ausgestaltung der Klauensicherung 5g und der Gegenanschläge 2g erreicht, dass nach dem Ausschütten der letzten Dosis der Pen nicht mehr aufgezogen werden kann, da die Gewindestange über den Formschluss zwischen Klauensicherung 5g und Gegenanschlägen 2g verdrehsicher gehalten wird. Ein weiteres Aufdosieren des Pens ist nicht mehr möglich, da die Gewindestange 5 verdrehsicher in der Führungshülse 3 gelagert ist und somit aufgrund der Klauenkupplung 2g, 5g ein Drehen des Dosierknopfes 1 bzw. der Drehhülse 2 verhindert wird.

Zum Einsetzen oder Eindrehen in die Injektionsvorrichtung kann eine Zweikammer-Ampulle 13 verwendet werden. Zum Abmischen wird die Ampulle 13 in den Pen hineingeschraubt, wodurch bei ausreichend weitem Hineinschrauben der Ampulle 13 in den Pen diese in Anlage an die Führungshülse 3 kommt und diese zusammen mit dem Dosierknopf 1 in proximale Richtung des Pens schiebt. Hierdurch wird der Dosierknopf 1 aus dem Pen herausgeschoben, was eine Einstellung oder ein Aufziehen des Pens überhaupt erst ermöglicht.

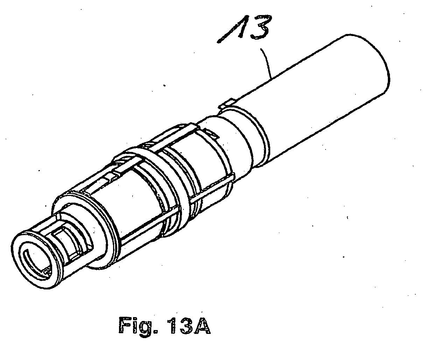

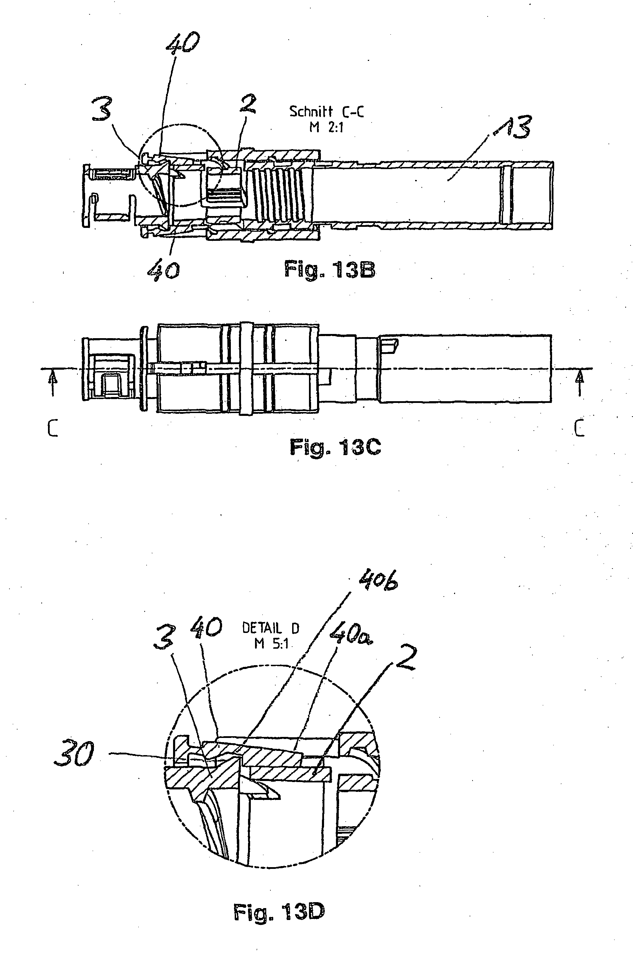

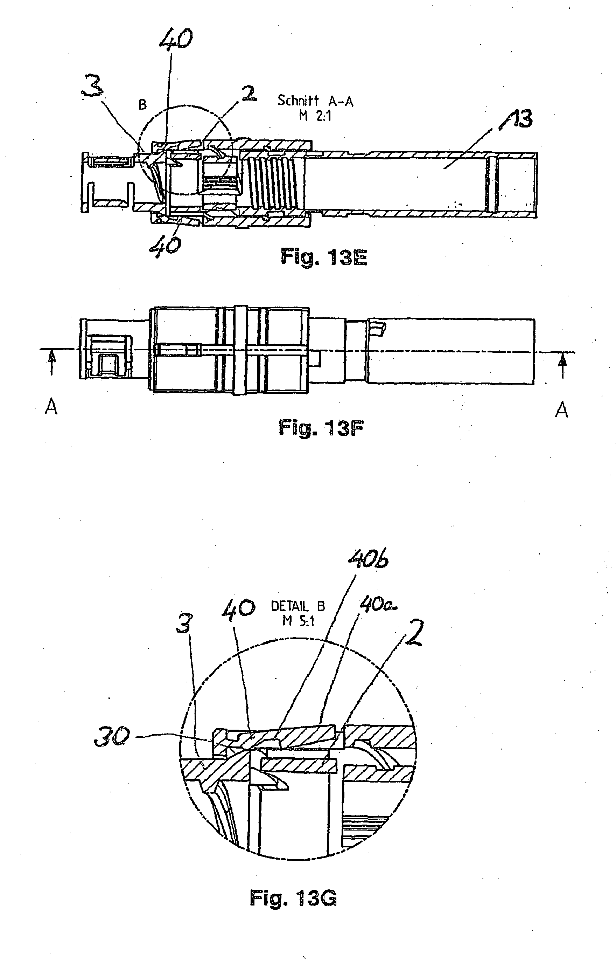

Beispielsweise kann, wie in den Figuren 13B und 13C und der Detailansicht in Figur 13D gezeigt, ein Rastring 40 oder Sperrring vorgesehen sein, dessen zwei gabelförmigen Sperrklinken 40a in entsprechende Vertiefungen der Drehhülse 2 hineinragen und eine Drehung der Drehhülse 2 verhindern. Da die Injektionsvorrichtung durch ein Drehen der Drehhülse 2 geladen wird, wird das Aufziehen oder Laden der Injektionsvorrichtung durch das Eingreifen des Rastrings 40 in die Drehhülse 2 verhindert.

Zum Entsperren des Rastrings 40 und der Drehhülse 2 wird die Ampullenhülse, welche zum Abmischen der Zwei-Kammer-Ampulle in den Pen eingeschraubt wird, eingedreht. Auf den letzten Millimetern, wie den letzten 1 bis 3 mm, beispielsweise den letzen ca. 2 Millimetern, des Einschraubvorganges, wird der Rastring 40 durch die Ampullenhülse von der Sperrposition in eine entsperrte Position gebracht, in welcher der Rastring 40 nicht mehr mit der Drehhülse 2 verrastet ist. Ist die Ampulle 13 ausreichend weit eingedreht, werden die beiden Sperrklinken 40 des Rast- oder Sperrrings 40, wie in den Figuren 13E bis 13G gezeigt, hinausgedrückt, indem auf den Innenseiten der beiden gabelförmigen Sperrklinken 40a angeordnete schräge Flächen 40b oder Gleitflächen relativ zu an der Führungshülse 3 ausgebildeten schrägen Flächen 30 oder Gleitflächen entlang gleiten, so dass der Rastring 40 außer Eingriff mit der Drehhülse 2 gelangt und die Drehhülse 2 für Drehbewegungen, zum Beispiel für Dosis- oder Dosiereinstellungen, freigegeben wird.

Zusätzlich ist auf der Gewindestange 5 noch eine Anzeigenhülse 6 vorgesehen, die fest, d.h. drehfest und axial verschiebesicher, mit der Gewindestange 5 verbunden ist. Auf der Außenseite der Anzeigenhülse 6 sind in umlaufender Richtung die noch auszuschüttenden Dosismengen aufgezeichnet Ein Sichtfenster 12 kann gebildet werden durch transparente Materialien oder Durchbrüche 12.3, 12.9, 12.7 in (von innen nach außen) Führungshülse 3, Ampullenhalter 9 und Gewindehülse 7.

Die Anzeigenhülse 6 ist bei abgemischter Ampulle 13 in diese eingeschoben (vorher nicht). Prinzipiell könnte die Anzeigenhülse 6 auch drehbar an dem hinteren Stopfen 13a angebracht sein, so dass die Anzeigenhülse 6 von der Mechanik des Pens zunächst entkoppelt ist und im Ampullenteil vorgesehen ist.

Da eine direkte Kopplung der Anzeigenhülse 6 mit der Gewindestange 5 besteht, kann die Anzeigenhülse 6 nicht verrutschen. Hierdurch kann es auch nicht zu einer Fehlanzeige kommen, selbst wenn der Pen zum Beispiel durch ein Herunterfallen einem starken Stoß ausgesetzt wird.

Die Führungshülse 3 dient auch als Sichtschutz, da das Fenster 12.3 in der Führungshülse 3 relativ zum Fenster 12.7 in der Gewindehülse 7 verschoben ist, bevor die Ampulle 13 eingeschraubt ist. Erst nach dem Einschrauben und Abmischen der Ampulle 13 wird das Fenster 12.3 der Führungshülse 3 in Deckung mit dem Fenster 12.7 der Gewindehülse 7 gebracht, so dass hierdurch die auf der Gewindestange 5 gelagerte Anzeigenhülse 6 sichtbar und ablesbar wird.

Der Auslöseknopf 11 wird durch ein Loch 10a in dem Gehäuse 10 positioniert und weist in Umlaufrichtung zwei Federarme 11a auf, welche den Auslöseknopf 11 radial nach außen weg von der Führungshülse 3 drücken. Die Federarme 11a beschreiben einen Radius, welcher kleiner als der Außenradius der Führungshülse 3 ist, so dass hierdurch die radial nach außen gerichtete Vorspannung des Auslöseknopfes 11 realisiert werden kann.

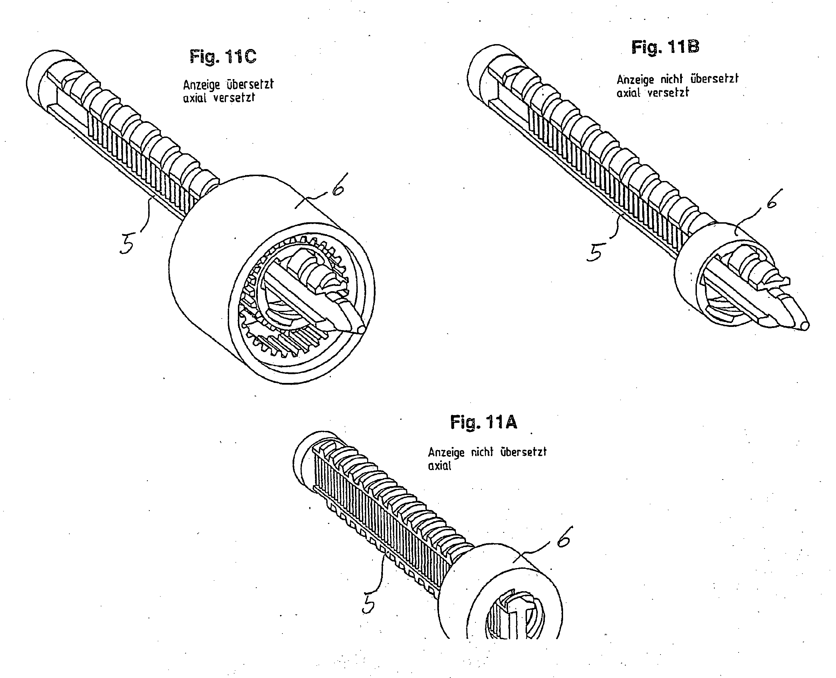

Die Anzeige 6 ist mit der Kolbenstange oder Gewindestange 5 in den in Fig. 11A und 11B gezeigten Ausführungsformen direkt gekoppelt und kann um diese ohne Selbsthemmung gedreht werden. Das Anzeigeelement 6 ist im Pen gegen axiale Verschiebung gesichert.

Die Kolbenstange 5 hat auf der Außenseite ein Gewinde oder Gewindeteilstück, in welches die Anzeige 6, oder bei Übersetzung der Anzeige 6 ein mit der Anzeige 6 gekoppeltes Übertragungselement, zum Beispiel ein Zahnrad oder wie in Fig. 11C gezeigt ein Zahnrad mit Innengewinde, eingreifen kann. Wird eine Übersetzung verwendet, so entsteht zwischen dem unmittelbar mit der Zahnstange gekoppelten Zahnrad und der Anzeige ein Zwischenraum, durch welchen zum Beispiel die Führungshülse 3 hindurchgeführt werden kann.

Wird anstatt der Zahnstange ein Drehmechanismus unter Verwendung einer Kolbenstange verwendet, so kann auch die Restmengenanzeige eingesetzt werden. Hierzu könnte das Restmengen-Anzeigelement 6 zum Beispiel auf der Kolbenstange 5 verdrehgesichert gelagert sein, so dass sich die Kolbenstange 5 während einer Ausschüttung durch die Restmengenanzeige 6 hindurchbewegt, die selbst axial verschiebegesichert in dem Pen gelagert ist.

Eine selbsthemmungsfreie Restmengenanzeige 6 kann durch eine geeignete Gewindesteigung realisiert werden, die materialabhängig ist und im Ausführungsbeispiel etwa 45° beträgt.

Vorzugsweise ist die Kopplung zwischen Restmengenanzeigeelement 6 und Zahnstange 5 so ausgelegt, dass bei vollständig hindurchgefahrener Zahnstange 5 das Restmengenanzeigeelement eine volle Drehung von 360° ausgeführt hat.

Bei einer Drehung von > 360° kann das Anzeigeelement 6 so ausgelegt sein, dass dieses mittels Außengewinde zusätzlich verschiebbar ist.

Denkbar wäre auch eine axial verschiebbare Restmengenanzeige 6, welche sich während des Hindurchfahrens der Zahnstange 5 in axiale Richtung des Pens bewegt, zum Beispiel durch einen Gewindeeingriff an der Außenseite der Restmengenanzeige 6 in ein Innengewinde im Gehäuse des Pens. Es kann zum Beispiel ein Pen mit konstanter voreingestellter Dosis verwendet werden, wobei die Restmengenanzeige zum Beispiel 14 maximal mögliche auszuschüttenden Einheiten anzeigt, die ausgehend von einem Anfangszustand auf 0 zurückgezählt werden.

## Patentansprüche

1. Injektionsvorrichtung mit einem Gehäuse (10), einer Führungshülse (3), die mit dem Gehäuse (10) verbunden und relativ zu dem Gehäuse (10) verdrehsicher gelagert ist, und einer Gewindestange (5), die verdrehbar und aufgrund einer Gewindekopplung axial verschiebbar ist, wobei die Gewindekopplung zwischen der Führungshülse (3) und der Gewindestange (5) besteht,
wobei die Gewindestange (5) eine Verdreh- oder Klauensicherung (5g) umfasst, welche in Ausnehmungen, Einbuchtungen, Einkerbungen, Nuten oder Klauen der Injektionsvorrichtung eingreift, wenn die Gewindestange (5) in eine distale Endposition innerhalb der Injektionsvorrichtung verschoben wurde, um eine Verdrehung der Gewindestange (5) und dadurch eine axiale Verschiebung der Gewindestange (5) relativ zur Injektionsvorrichtung zu verhindern.

2. Injektionsvorrichtung nach Anspruch 1, wobei die Verdreh- oder Klauensicherung (5g) an dem proximalen Ende der Gewindestange (5) angebracht ist.

3. Injektionsvorrichtung nach einem der vorhergehenden Ansprüche, wobei die Verdreh- oder Klauensicherung (5g) an einer Erweiterung (5f) der Gewindestange (5) angebracht ist.

4. Injektionsvorrichtung nach einem der vorhergehenden Ansprüche, wobei die Verdreh- oder Klauensicherung (5g) zumindest ein, insbesondere vier Verdrehsicherungselemente oder Klauen aufweist.

5. Injektionsvorrichtung nach dem vorhergehenden Anspruch, wobei die Verdrehsicherungselemente als Stege an der Gewindestange (5) ausgebildet sind.

6. Injektionsvorrichtung nach einem der zwei vorhergehenden Ansprüche, wobei die Verdrehsicherungselemente oder Klauen zumindest leicht und/oder teilweise verformbar sind.

7. Injektionsvorrichtung nach einem der drei vorhergehenden Ansprüche, wobei die Verdrehsicherungselemente oder Klauen federnd gelagert sind.

## Claims

1. An injection device comprising a housing (10), a guide sleeve (3) which is connected to the housing (10) and mounted non-rotatably relative to the housing (10), and a threaded rod (5) which is rotatable and axially displaceable by virtue of a thread coupling, wherein the thread coupling is between the guide sleeve (3) and the threaded rod (5),
wherein the threaded rod (5) includes a rotation-preventing or claw securing means (5g) which engages into openings, recesses, notches, grooves or claws (2g) of the injection device when the threaded rod (5) has been displaced into a distal end position within the injection device to prevent rotation of the threaded rod (5) and thereby axial displacement of the threaded rod (5) relative to the injection device.

2. An injection device according to claim 1 wherein the rotation-preventing or claw securing means (5g) is disposed at the proximal end of the threaded rod (5).

3. An injection device according to one of the preceding claims wherein the rotation-preventing or claw securing means (5g) is disposed at an enlargement (5f) of the threaded rod (5).

4. An injection device according to one of the preceding claims wherein the rotation-preventing or claw securing means (5g) has at least one and in particular four rotation-preventing elements or claws.

5. An injection device according to the preceding claim wherein the rotation-preventing elements are in the form of limbs on the threaded rod (5).

6. An injection device according to one of the two preceding claims wherein the rotation-preventing elements or claws are at least easily and/or partially deformable.

7. An injection device according to one of the three preceding claims wherein the rotation-preventing elements or claws are resiliently mounted.

## Revendications

1. Dispositif d'injection avec un boîtier (10), une douille de guidage (3), qui est reliée au boîtier (10) et montée de manière bloquée en rotation par rapport au boîtier (10), et une tige filetée (5), qui est mobile en rotation et axialement sur la base d'un accouplement fileté, dans lequel l'accouplement fileté est présent entre la douille de guidage (3) et la tige filetée (5)
dans lequel la tige filetée (5) comprend une protection contre la torsion ou à griffes (5g), laquelle s'insère dans des évidements, cavités, encoches, rainures ou griffes du dispositif d'injection, lorsque la tige filetée (5) a été déplacée dans une position d'extrémité distale à l'intérieur du dispositif d'injection, afin d'empêcher une torsion de la tige filetée (5) et de ce fait un déplacement axial de la tige filetée (5) par rapport au dispositif d'injection.

2. Dispositif d'injection selon la revendication 1, dans lequel la protection contre la torsion ou à griffes (5g) est montée sur l'extrémité proximale de la tige filetée (5).

3. Dispositif d'injection selon l'une quelconque des revendications précédentes, dans lequel la protection contre la torsion ou à griffes (5g) est disposée à une extension (5f) de la tige filetée (5).

4. Dispositif d'injection selon l'une quelconque des revendications précédentes, dans lequel la protection contre la torsion ou à griffes (5g) présente au moins un, en particulier quatre, éléments de protection contre la torsion ou griffes.

5. Dispositif d'injection selon la revendication précédente, dans lequel les éléments de protection contre la torsion sont conçus comme entretoises sur la tige filetée (5).

6. Dispositif d'injection selon l'une quelconque des deux revendications précédentes, dans lequel les éléments de protection contre la torsion ou les griffes peuvent se déformer au moins légèrement et/ou partiellement.

7. Dispositif d'injection selon l'une quelconque des trois revendications précédentes, dans lequel les éléments de protection contre la torsion ou les griffes sont placés de manière élastique.
